# EUROPÄISCHE PATENTSCHRIFT

(11) **EP 1 098 676 B1**
(45) Veröffentlichungstag und Bekanntmachung des Hinweises auf die Patenterteilung: **06.11.2002**
(21) Anmeldenummer: 00951394.6
(22) Anmeldetag: 18.07.2000
(51) Int. Cl.: A61N 1/40, A61N 2/02

(54) **MAGNETFELDAPPLIKATOR ZUR AUFHEIZUNG VON MAGNETISCHEN ODER MAGNETISIERBAREN SUBSTANZEN ODER FESTKORPERN IN BIOLOGISCHEM GEWEBE**
MAGNETIC FIELD APPLICATOR FOR HEATING MAGNETIC OR MAGNETIZABLE SUBSTANCES OR SOLIDS IN BIOLOGICAL TISSUE
APPLICATEUR DE CHAMP MAGNETIQUE POUR LE CHAUFFAGE DE SUBSTANCES OU DE CORPS SOLIDES MAGNETIQUES OU MAGNETISABLES DANS DES TISSUS BIOLOGIQUES

(30) Priorität: 07.08.1999 DE 19937493
(43) Veröffentlichungstag der Anmeldung: 16.05.2001
(73) Patentinhaber: MFH Hyperthermiesysteme GmbH, 12247 Berlin (DE)
(72) Erfinder: FEUCHT, Peter, D-10827 Berlin (DE)
(74) Vertreter: Liebl, Thomas, Dipl.-Ing.
(86) Internationale Anmeldenummer: EP0006835
(87) Internationale Veröffentlichungsnummer: WO01010500

(56) Entgegenhaltungen:
- EP-A- 0 913 167
- FR-A- 2 508 802
- US-A- 5 197 940

## Beschreibung

Die Erfindung betrifft einen Magnetfeldapplikator zur Aufheizung von magnetischen oder magnetisierbaren Substanzen oder Festkörpern in biologischem Gewebe nach dem Oberbegriff des Anspruchs 1.

Krebserkrankungen werden in allgemein bekannter Weise durch eine chirurgische Entfernung, eine Chemotherapie, eine Bestrahlungstherapie oder eine Kombination dieser Methoden behandelt. Jede dieser Methoden unterliegt gewissen Beschränkungen: Eine operative Entfernung des Tumors ist besonders bei fortgeschrittenen Stadien, nach Metastasierung, bei Lage des Tumors in Nachbarschaft zu kritischen Körperarealen oder diffusem Tumorwachstum mit ungenauer Lokalisation nicht möglich oder bietet nur geringe Heilungschancen. Deshalb wird die chirurgische Intervention im allgemeinen kombiniert mit Strahlentherapie und Chemotherapie. Erstere kann nur so genau sein, wie die Lokalisation des Tumors durch bildgebende Verfahren unter weitestgehender Aussparung bzw. Schonung des gesunden Gewebes. Chemotherapeutische Mittel wirken dagegen systemisch, d. h. auf den ganzen Körper. Hier ist in der Regel die Knochenmarkstoxizität bzw. die Unspezifität der Therapie limitierend. Unerwünschte Nebenwirkungen sind deshalb bei allen diesen Therapieverfahren nach dem Stand der Technik unvermeidlich und schädigen in der Regel auch gesundes Gewebe.

Als weitere Modalität hat in den letzten Jahren zunehmend die Hyperthermie an Bedeutung gewonnen, bei der das Tumorgewebe auf Temperaturen über 41°C aufgeheizt wird und so in Kombination mit Chirurgie, Strahlentherapie und Chemotherapie der Behandlungserfolg, d. h. lokale Kontrolle, z. T. sogar Überleben, verbessert werden kann. Im Temperaturbereich zwischen 41 und 46°C kommt es vom Körper unterstützt zu einem kontrollierten und eher langsamen Abbau des Tumorgewebes. Dieser Prozess wird Hyperthermie genannt, während bei höheren Temperaturen ab 47°C eine akute Zellzerstörung je nach Temperatur als Nekrose, Koagulation oder Karbonisation stattfindet und deshalb als Thermoablation bezeichnet wird. Hyperthermiesysteme nach dem Stand der Technik sind entweder nur zur vorstehend genannten Hyperthermie oder nur zur Thermoablation geeignet.

Ein Problem bei der Hyperthermie allgemein besteht darin, dass in der Regel keine genau lokalisierbare und vor allem homogene Erwärmung einer Zielregion des Körpers nach Stand der Technik möglich ist. Unter bestimmten physiologischen Bedingungen (z. B. Sauerstoffunterversorgung, niedriger pH) im Tumor sind zwar Krebszellen empfindlicher gegenüber Hyperthermie, jedoch trifft dies nur in wenigen Fällen zu. Hyperthermie per se ist nicht wirksamer auf Tumorzellen im Vergleich zu Normalgewebe. Deshalb ist die Begrenzung der Erwärmung auf die ärztlich indizierte Region (die nicht unbedingt nur auf die Geschwulst beschränkt sein muss) von besonderer Bedeutung und nach dem Stand der Technik nicht realisiert.

Nach dem Stand der Technik werden E-Feld dominante Systeme verwendet, die elektromagnetische Wellen in der Regel im Megahertz-Bereich von Dipol-Antennen oder anderen Antennen-Bauformen oder Antennen-Arrays abstrahlen, die zur regionalen oder lokalen Hyperthermie verwendet werden. Dabei nutzt man entweder das elektrische Feld einzelner E-Feld Applikatoren für die sogenannte interstitielle Hyperthermie oder die Interferenz von Antennen-Arrays für die Tiefenhyperthermie. Gemeinsame Schwierigkeit aller dieser E-Feld dominanten Systeme ist, dass die Leistungsabsorption nur durch die aufwendige Steuerung des E-Feldes erzielt werden kann und dass die Erwärmung abhängig ist von der elektrischen Leitfähigkeit der jeweiligen Zielgewebe, die naturgemäß sehr heterogen ist, wodurch sich eine ungleichmäßige Erwärmung auch bei homogener Abstrahlung des E-Feldes ergibt. Besonders an den Übergangsstellen von Körperregionen sehr unterschiedlicher elektrischer Leitfähigkeit kommt es deshalb zu Leistungsüberhöhungen, den sogenannten "hot spots", die zu Schmerzen und Verbrennungen des Patienten führen können. Die Folge ist eine meist vom Patienten erzwungene Reduktion der insgesamt abgegebenen Leistung, so dass dadurch auch in der Zielregion nicht die erforderliche Temperatur zur irreversiblen Schädigung des Tumorgewebes (41-42°C) erreicht wird und deshalb der Therapieerfolg ausbleibt. Durch die Interferenz von Dipol-Arrays ist außerdem nur die Erzeugung eines zweiten E-Feld Maximums in tiefergelegenen Körperarealen möglich. Die größte Leistungsabsorption findet aus physikalischen Gründen immer an der Oberfläche des Körpers, d. h. bei maximalem Radius statt. Hinzu kommt, dass sich unter Hyperthermie häufig die Durchblutung sowohl des Tumor- als auch des Normalgewebes verändert und diese Veränderung mittels E-Feld dominanter Systeme von außen aufgrund der eher geringen Steuerungsmöglichkeiten des Feldes nicht kompensiert werden kann.

Weitere Verfahren nach dem Stand der Technik sind Ultraschall vorzugsweise zur Thermoablation und interstitielle Mikrowellenapplikatoren. Letztere haben aufgrund der Frequenz eine geringe Eindringtiefe und können deshalb nur in Form von interstitiellen Antennen eingesetzt werden. Darüberhinaus wird Infrarot für die Ganzkörperhyperthermie eingesetzt, sowie extrakorporale Systeme zur Aufheizung von Körperflüssigkeiten.

Weiter ist ein Hyperthermieverfahren zur Therapie von Prostatakrebs bekannt (US 5 197 940), bei dem im Tumorbereich "Thermoseeds" eingebracht sind, die aus magnetischem, insbesondere ferromagnetischem oder aus magnetisierbarem Material bestehen oder ein solches enthalten. Diese Thermoseeds haben eine typische Größe von mehreren Zentimetern Länge und einen Durchmesser im Millimeterbereich. Ersichtlich ist es erforderlich, solche Thermoseeds in aufwendiger Weise chirurgisch zu implantieren. Diese Thermoseeds werden bei einer Behandlung mit einem außerhalb eines Patienten erzeugten Magnetwechselfeld beaufschlagt, wobei durch an sich bekannte Hystereseeffekte Wärme in den Thermoseeds als Hyperthermie entsteht.

Die Erwärmung solcher Seeds geschieht allerdings nach dem Prinzip der "heißen Quellen", d. h. während sich die Seeds aufheizen, fällt die Temperaturen in der Umgebung des Seeds exponentiell ab, so dass in der klinischen Anwendung der Abstand zwischen den Seeds nicht größer als 1 cm sein darf. Bei größeren oder ungleichmäßigen Abständen entsteht eine thermische Unterdosierung, die ebenfalls den Therapieerfolg verhindern kann. Gerade bei größeren Tumoren entsteht so die Notwendigkeit einer sehr engen Implantation der Seeds, wodurch die Methode chirurgisch aufwendig und für den Patienten belastend ist. Abgesehen vom geringen Abstand müssen die Seeds auch für optimale Leistungsaufnahme parallel zum magnetischen Wechselfeld ausgerichtet sein. Eine Überhitzung wird durch die Curie-Temperatur bei sogenannten selbstregulierenden Thermoseeds verhindert, bei denen nach Erreichen der Curie-Temperatur das Ferrit in einen nicht-magnetisierbaren Zustand übergeht und dann keine weitere Leistungsaufnahme mehr erfolgt.

Als Magnetfeldapplikator für das Magnetwechselfeld ist hier eine Magnetspule eines Schwingkreises verwendet, in derem Achsbereich die Körperregion eines Patienten mit den implantieren Thermoseeds einbringbar ist. Konkret sind hier Luftspulen verwendet, in deren mittleren Bereichen ein Patient bei einer Behandlung auf einer nicht magnetiesierbaren Abstützplatte sitzt.

Nachteilig an der interstitiellen Hyperthermie mit Thermoseeds ist der hohe chirurgische Aufwand und die hohe Invasivität der Methode, das Risiko einer nicht exakten Ausrichtung oder Positionsänderung der Seeds und dem damit verbundenen Risiko thermischer Unterdosierung sowie eine Begrenzung der Methode auf Tumore kleinerer Ausdehnung.

Bei einem weiteren bekannten Hyperthermieverfahren (WO 97/43005) zur Tumortherapie werden magnetisierbare Mikrokapseln vorgeschlagen, die durch den Blutstrom in den Tumorbereich gelangen. Damit sollen unter anderem chirurgische Implantationen von magnetisierbaren Elementen vermieden werden, da bei Implantationen neben den Beschwernissen für einen Patienten auch die Gefahr besteht, dass bei einem Schnitt in den Tumor entartete Tumorzellen in gesundes Gewebe streuen. Zur Befeldung wird ein lineares Magnetwechselfeld verwendet mit einer Frequenz im Bereich zwischen 10 kHz bis 500 kHz. Die Mikrokapseln sollen in Verbindung mit einem hochmagnetisierbaren Material verwendet werden, so dass die für die Befeldung erforderliche Stärke des Magnetwechselfeldes hinsichtlich des apparativen Aufbaus der erforderlichen Kühlsysteme sowie der elektrischen Energieversorgung beherrschbar werden. Ein konkreter apparativer Aufbau ist jedoch nicht angegeben.

In einem weitgehend ähnlichen bekannten Hyperthermieverfahren (EP 0 913 167 A2) werden zur Befeldung rotierende Magnetfelder mit einer Frequenz im Bereich größer 10 kHz verwendet. Zur Erzeugung des hier verwendeten rotierenden Magnetwechselfeldes ist lediglich skizzenhaft und schematisch ein gattungsgemäßer Magnetfeldapplikator angegeben. Der Magnetfeldapplikator umfasst ein Magnetjoch mit jeweils zwei gegenüberliegenden, durch einen Befeldungsspalt beabstandeten Polschuhen und zwei jeweils diesen Polschuhen zugeordneten Magnetspulen. Konkret ist ein rechteckiges Magnetjoch gezeigt, bei dem ausgehend von der Mitte jedes Jochschenkels ein Polschuh auf die Mitte des Rechtecks zugerichtet ist, wodurch dort ein Befeldungsraum gebildet wird. An den Polschuhen sind Zylinderspulen angebracht, die jeweils gegenüberliegend miteinander und mit einer zugeordneten Kondensatoranordnung zu einem Schwingkreis verbunden sind.

Diese schematische Darstellung eines Magnetfeldapplikators zur Durchführung des vorstehenden Hyperthermieverfahrens führt noch zu keiner, über ein Experimentierstadion hinausgehenden konstruktiven Serienlösung, wie sie hinsichtlich günstiger Herstellungs- und Betriebskosten, eines geringen Einbauraums mit geringer Streufeldbelastung und eines optimalen therapeutischen Effekts zur Verwendung im Krankenhausbetrieb erforderlich ist.

Aufgabe der Erfindung ist es daher, einen Magnetfeldapplikator zur Aufheizung von magnetischen oder magnetisierbaren Substanzen oder Festkörpern in biologischem Gewebe zu schaffen, der die vorstehenden Forderungen hinsichtlich einer Serienlösung für den Krankenhausbetrieb oder eines anderweitigen, ggf. industriellen Einsatzes erfüllt.

Diese Aufgabe wird mit den Merkmalen des Anspruchs 1 gelöst.

Gemäß Anspruch 1 bestehen das Magnetjoch und die Polschuhe aus zusammengesetzten und zusammenmontierten Ferritbausteinen. Zudem ist das Magnetjoch in einer M-Form als Drei-Schenkel-Anordnung auf mit zwei beabstandeten, parallelen Vertikaljochteilen gleicher Geometrie und mit zwei dazwischen angeschlossenen Querjochteilen ausgebildet, an denen im mittleren Bereich die aufeinander zugerichteten Polschuhe mit den Magnetspulen angeordnet sind. Ein Querjochteil mit zugeordneter Magnetspule ist als Baueinheit relativ zum anderen Querjochteil zur Einstellung der Befeldungsspaltweite verstellbar ausgeführt. Vorteilhaft wird hier der magnetische Rückschluss zu beiden Seiten auf zwei gleich lange Wege aufgeteilt, die die gleiche Geometrie haben. Die Mechanik für die Relatiwerstellung wenigstens eines Querjochteils ist gegenüber einem C-Magnetjoch einfacher ausführbar, da die Vertikaljochteile als beidseitige Abstützungen nützbar sind.

Für die Hyperthermie, insbesondere mit magnetischen Flüssigkeiten, werden Wechselfeldstärken von ca. 15 bis 20 kA/m bei ca. 50 bis 100 kHz benötigt. Bei einem Befeldungsvolumen von 8 bis 30 l sind dabei von einer Hyperthermieanlage Wirkleistungen von ca. 18 kW bis 80 kW aufzubringen. Diese Energie muss als Hochfrequenz erzeugt werden und dann als Wärme wieder abgeführt werden, da für die Hyperthermie im Körper eines Patienten nur einige Watt in der magnetischen Flüssigkeit entstehen.

Mit der im Patentanspruch 1 beanspruchten Anordnung ist es möglich, das befeldete Volumen sowie Streufelder vorteilhaft klein zu halten und weitgehend auf einen zu therapierenden Bereich im Körper eines Patienten zu begrenzen, so dass der erforderliche Energieaufwand und Aufwand für den Wärmetransport reduzierbar ist. Dazu tragen insbesondere ein Magnetjoch und Polschuhe aus Ferritbausteinen sowie die Form des Magnetjochs bei, wodurch unerwünschte Überhöhungen der Flussdichten in Verbindung mit großen Verlusten wesentlich reduzierbar sind.

Die Verwendung von Ferritbausteinen ermöglicht in Verbindung mit der hohen Wechselfrequenz von ca. 50 bis 100 kHz eine vorteilhafte Begrenzung des Befeldungsvolumens, wobei im Ferritvolumen etwa nur 1/2000 der Energie bewegt wird, die ein äquivalentes Luftvolumen hätte. Diesem erheblichen Vorteil steht gegenüber, dass die Ferritbausteine jedoch verlustbehaftet sind, wobei z. B. eine Verdopplung der Flussdichte im Arbeitsbereich bereits 5 bis 6-fache Verluste bringen kann. Es werden daher nachstehend geeignete Maßnahmen angegeben, um die Flussdichten klein zu halten und insbesondere unerwünschte Flussdichteerhöhungen zu vermeiden oder wenigstens stark zu reduzieren.

Ferrite sind keramikartige Bausteine, die sich nicht in beliebigen Gestalten mit vertretbarem Aufwand herstellen lassen, insbesondere nicht in der Gesamtgestalt des hier verwendeten Magnetjochs. Daher wird erfindungsgemäß vorgeschlagen, das Magnetjoch aus Ferritbausteinen zusammenzusetzen, wobei an den Übergangsstellen Störungen eines möglichst gleichmäßigen Flussverlaufes auftreten können. Vorteilhafte Lösungen zur Beherrschung dieser Probleme sind weiter unten angegeben.

Der erfindungsgemäße Magnetfeldapplikator ist gleichermaßen geeignet sowohl für die Durchführung von Hyperthermien als auch für Thermoablationsverfahren. Darüberhinaus ist der erfindungsgemäße Magnetfeldapplikator geeignet zur Erwärmung auch anderer Substanzen oder Festkörper für medizinische Anwendungen außerhalb der Krebstherapie. Unter letzteren werden alle erwärmungsbedingten medizinischen Anwendungen verstanden, wie z. B. wärmeinduzierte Implantat- bzw. Stentregenerierung, Implantat- bzw. Stentoberflächenaktivierung, Aufheizung von nicht durch Krebs erkrankten bzw. entzündlichen Körperregionen zu Therapiezwecken, die erleichtere Konstrastmittelverteilung oder -verbesserung mittels Magnetwechselfeldanregung superparamagnetischer Kontrastmittel, das Einschalten molekularbiologischer, zellbiologischer und entwicklungsphysiologischer Vorgänge mittels Anregung Magnetcarriergestützter Gentransfersysteme, Liganden, Rezeptoren, Transmittern, sonstigen Signalmolekülen sowie die Auslösung von Stoffwechselvorgängen und endokrinen Prozessen.

Dazu wird nach Anspruch 2 eine Baueinheit aus einem unteren Querjochteil und dem zugeordneten Polschuh mit Magnetspule ortsfest angebracht. Auf diesem ortsfesten Polschuh kann dann beispielsweise ein Patientenschlitten mit Patientenlager und Schlittenpositionsanzeige aus Kunststoffmaterial angebracht werden, wobei dann der Patient bei einer Einstellung der Befeldungsspaltweite nicht mehr bewegt werden muss. Gegenüber dieser ortsfesten Baueinheit ist dann ein Portal aus den beiden Vertikaljochteilen aus dem oberen Querjochteil mit dem zugeordneten Polschuh mit Magnetspule mittels einer Vertikalverstelleinrichtung zur Einstellung der Befeldungsspaltweite verstellbar.

Eine Vertikalverstelleinrichtung kann nach Anspruch 3 als einfacher Linearantrieb ausgeführt werden, der vorzugsweise jeweils an einem Vertikalmagnetjochteil angreift. Beispielsweise kann ein selbsthemmender Spindeltrieb eingesetzt werden, wodurch die Gesamtanordnung sehr sicher ausführbar ist ohne die Gefahr, dass schwere Magnetjochbauteile durch Fehler in der Verstelleinrichtung zu einer Gefährdung eines Patienten führen.

In einer vorteilhaften Weiterbildung nach Anspruch 4 kann das Magnetjoch in einer Tragkonstruktion gehalten sein, in der zudem Kühlluft zuführbar und leitbar ist, welche die Kühlluftspalte der Ferritbausteine zur Wärmeabfuhr durchströmt.

Je nach den Gegebenheiten und den speziellen Erfordernissen kann nach Anspruch 5 der Befeldungsspalt und damit das Befeldungsvolumen seitlich durch Feldbegrenzungsspulen und/oder durch Schottwände begrenzt sein.

Grundsätzlich kann der erfindungsgemäße Magnetfeldapplikator für eine genau lokalisierte und berührungsfreie Hyperthermie an allen möglichen zu befeldenden Geweben, Körpern, Gegenständen und Massen unter Verwendung eingebrachter magnetischer und/oder magnetisierbarer Substanzen zu geeigneten Zwecken verwendet werden. Eine bevorzugte Anwendung des Magnetfeldapplikators liegt nach Anspruch 6 jedoch im medizinischen Bereich, insbesondere in der Krebstherapie, wobei vorzugsweise als magnetische Substanz eine Flüssigkeit mit magnetisierbaren Nanoteilchen verwendet ist. Ein Tumorbereich soll dann auf Temperaturwerte über ca. 41°C lokal erhitzbar sein.

Nach Anspruch 7 werden dazu Magnetwechselfelder mit Magnetfeldstärken von ca. 10 bis 15 kA/m und Frequenzen von ca. 50 bis 100 kHz eingesetzt. In Verbindung mit dem vorstehend beanspruchten Magnetfeldapplikator werden dann die erforderlichen Temperaturen für eine Tumortherapie erreicht. Für eine Thermoseed-Anwendung des Magnetfeldapplikators reichen bereits 1 bis 2 kA/m aus. Je nach den vorliegenden Gegebenheiten können auch Frequenzen in einem weiteren Frequenzbereich von 20 bis 500 kHz geeignet sein.

Bei einer grundsätzlich möglichen Anordnung mit Zylinderspulen um die Polschuhe treten in deren letzter Windung zum Befeldungsspalt durch induktive Erwärmung Temperaturerhöhungen auf mit erforderlichen Maßnahmen bei der Wärmeabfuhr. Die scheibenförmige Spulenausführung mit wenigstens einer schneckenförmigen Windung in Verbindung mit dem Magnetspule/Polschuh-Spalt nach Anspruch 8 führt dagegen zu geringeren Flussdichten auf der umlaufenden Kante der jeweils zugeordneten Polschuhe. Unerwünschte Überhöhungen der Flussdichten sind reduzierbar.

In einer konkreten Ausführungsform nach Anspruch 9 sollen die Magnetspulen eine oder mehrere Windungen aufweisen, die schneckenförmig verlaufen und die aus verseilten Kupfer-Litzendrähten hergestellt sind, um Wirbelstromverluste möglichst klein zu halten.

In einer besonders vorteilhaften Ausbildung werden nach Anspruch 10 die Polschuhe zylindrisch bzw. in einer Draufsicht kreisrund ausgeführt, wobei sie mit gegeneinanderweisenden, parallel ausgerichteten Polschuhkreisflächen im Abstand des Befeldungsspalts gegenüberliegen. Entsprechend sind dann die Magnetspulen kreisringförmig ausgebildet. Dies führt zu einer Vergleichmäßigung des Magnetflusses mit einer Reduzierung von Erwärmungen, die ansonsten an räumlichen Ecken und Kanten erhöht auftreten würden.

Energetisch und flusstechnisch besonders günstige Verhältnisse ergeben sich nach Anspruch 11, wenn die scheibenförmige Magnetspule möglichst nahe am Befeldungsspalt angeordnet ist, insbesondere in einer flächenbündigen Anordnung bezüglich der jeweils zugeordneten Polschuhendfläche. Eine weitere Optimierung wird erreicht, wenn dabei der Magnetspule/Polschuh-Spalt in einer Größe von etwa 1/10 des Polschuhdurchmessers (0,07 bis 0,1-fach) liegt und die umlaufende Kante der jeweiligen Polschuhendfläche abgerundet ist. Damit werden schädliche Flussdichteüberhöhungen stark reduziert.

Gemäß Anspruch 12 soll der Polschuhdurchmesser größer als die Befeldungsspaltweite sein. Dadurch werden Streufelder außerhalb der Polschuhe bzw. des Befeldungsvolumens reduziert, wodurch die Flussdichte in den Ferritbausteinen und damit die Verluste im Ferritmaterial relativ klein gehalten werden können. Bei Polschuhen mit relativ kleinem Querschnitt würden diese Verluste in den Ferritbausteinen überproportional steigen.

Gemäß Anspruch 13 ist das Magnetjoch aus quaderförmigen Ferritbausteinen zusammengesetzt, deren Flächen zur Schaffung gleichmäßiger Übergänge plan-parallel geschliffen sind, wobei ggf. äußere Sinterschichten entfernt sind. Entsprechend sind die runden Polschuhe aus keilförmigen Ferritbausteinen wie aus Tortenstücken zusammengesetzt, wobei auch hier benachbarte Flächen plan-parallel geschliffen sind.

Zur Senkung der Wirbelstromverluste wird mit Anspruch 14 vorgeschlagen, die quaderförmigen Ferritbausteine aus aneinandergereihten Ferritplatten herzustellen und jeweils durch Isolations-/Kühlungs-Spalte beabstandet zu halten. Im montierten Zustand sind diese Ferritplatten längs des Magnetflusses ausgerichtet. Zur Herstellung einstückiger Ferritbausteine aus den Ferritplatten werden diese nach Anspruch 15 mittels Separatoren aus Kunststoff auf Abstand gehalten und miteinander über die Separatoren verklebt.

Ähnlich werden nach Anspruch 16 die keilförmigen Ferritbausteine zur Bildung der Polschuhe hergestellt, wobei eine rohrförmige mittlere Aussparung offengelassen wird, durch die Kühlluft einleitbar ist. Zur Verklebung der Ferritplatten wird vorzugsweise ein temperaturbeständiger Zwei-Komponenten-Klebstoff verwendet.

Die Spalte zwischen den Ferritplatten dienen sowohl der elektrischen Isolation als auch der Kühlung, indem durch die Spalte Kühlluft geblasen wird. Die Kühlung ist erforderlich, da trotz geringer Leitfähigkeit der Ferrite relativ große Wirbelströme entstehen und die dadurch erzeugte Wärme abgeführt werden muss. Eine Flüssigkeitskühlung wäre zwar effektiver, ist aber wegen der Isolationsforderungen nicht realisierbar. Eine Ölkühlung ist wegen der Brennbarkeit von Ölen mit Gefahren verbunden und vergleichbare nichtbrennbare Flüssigkeiten enthalten regelmäßig Giftstoffe. Allgemein wäre bei einer Flüssigkeitskühlung das Dichtproblem insbesondere bei einem beweglichen Jochteil in Verbindung mit den übrigen technischen Schwierigkeiten nur mit hohem Aufwand lösbar.

Einerseits wird, wie weiter oben angegeben, der Magnetfluss an Übergangsstellen dadurch gesteuert, dass einerseits herstellungsbedingte, magnetisch inaktive Sinterschichten von etwa 0,1 bis 0,2 mm entfernt werden und zudem magnetisch leitende Flächen plan-parallel geschliffen werden. Wegen der hohen Ferrit-Permeabilität wirken sich kleinste Unebenheiten aus, so dass eine Flusssteuerung mit Zwangsluftspalten gemäß Anspruch 17 zweckmäßig ist. Insbesondere ist dies mit Zwangsspalten von 2 bis 3 mm an den Übergangsstellen zwischen den beweglichen Querjochteilen und angrenzenden Vertikaljochteilen und/oder an Übergangsstellen zwischen Querjochteilen und den Polschuhen vorteilhaft. Im Bereich solcher relativ breiter Zwangsspalte kann je nach den Gegebenheiten eine Sinterschicht zur Reduzierung der Herstellungskosten an einem Ferritbaustein verbleiben.

Anhand einer Zeichnung wird die Erfindung näher erläutert.

Es zeigen:
- Fig. 1: eine schematische Schnittansicht durch einen Magnetfeldapplikator,
- Fig. 2: eine schematische Draufsicht auf den Magnetfeldapplikator der Fig. 1,
- Fig. 3: eine schematische Seitenansicht des Magnetfeldapplikators der Fig. 1,
- Fig. 4: eine Draufsicht auf einen Polschuh mit keilförmigen Ferritbausteinen,
- Fig. 5: eine Seitenansicht des Polschuh der Fig. 4,
- Fig. 6: eine schematische, perspektivische und vergrößerte Darstellung des Aufbaus quaderförmiger Ferritbausteine,
- Fig. 7: eine schematische und vergrößerte Darstellung eines Übergangsbereiches zwischen einem Vertikaljochteil und einem Querjochteil, und
- Fig. 8: eine schematische Seitenansicht einer flächenbündig mit einer Polschuhendfläche angeordneten Magnetspule.

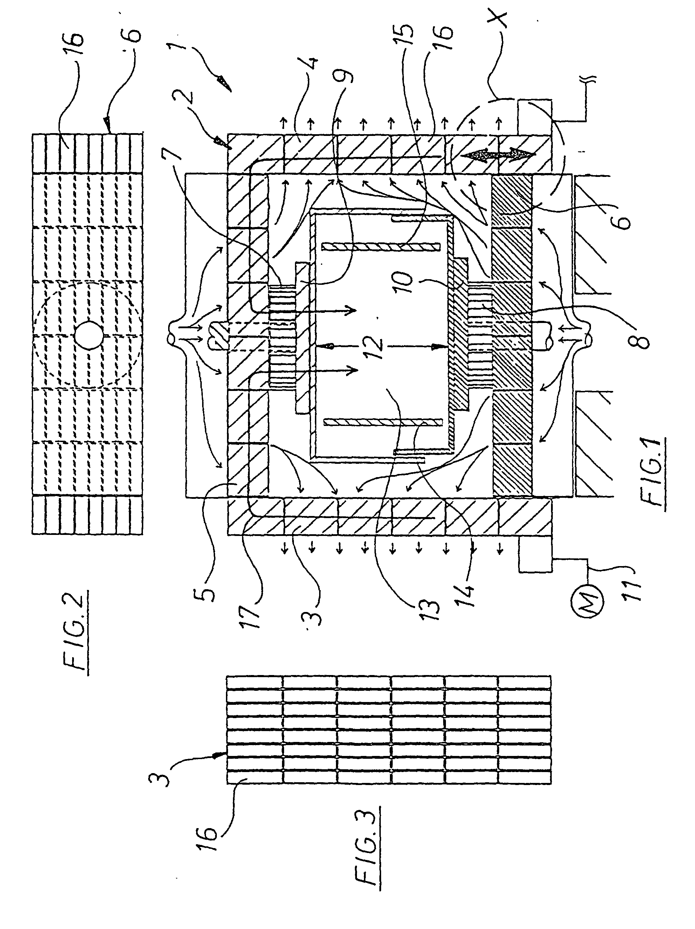

In der Fig. 1 ist schematisch ein Magnetfeldapplikator 1 für die Hyperthermie dargestellt, in den ein zu befeldender Körper, in den eine magnetische oder magnetisierbare Substanz oder Festkörper einbringbar ist, bestrahlbar ist. Als zu befeldender Körper eignet sich insbesondere ein Tumorbereich in einem menschlichen Körper, in dem eine Flüssigkeit mit z. B. magnetischen Nanoteilchen eingebracht ist, wobei der Tumorbereich auf Temperaturwerte vorzugsweise von über ca. 41°C erhitzbar ist.

Der Magnetfeldapplikator 1 umfasst ein Magnetjoch 2, das in einer M-Form als Drei-Schenkel-Anordnung ausgebildet ist und zwei beabstandete, parallele Vertikaljochteile 3, 4 sowie zwei dazwischen angeschlossene Querjochteile 5, 6 aufweist.

Eine Baueinheit aus unterem Querjochteil 6 und diesem zugeordneten unteren Polschuh 8 mit unterer Magnetspule 10 ist ortsfest angebracht. Demgegenüber kann ein Portal aus den beiden Vertikaljochteilen 3, 4, dem angeschlossenen oberen Querjochteil 5 sowie diesem zugeordneten oberen Polschuh 7 mit oberer Magnetspule 9 mittels eines hier lediglich schematisch dargestellten, selbsthemmenden Spindelantriebs 11 zur Einstellung der Befeldungsspaltweite des Befeldungsspaltes 12 verstellt werden.

Der Fig. 1 kann weiter entnommen werden, dass der Befeldungsspalt 12 durch Schottwände 14, 15 begrenzt ist, die einen Einschubraum 13 umgrenzen. Die Schottwände 14, 15 können dabei gegeneinander vertikal verstellbar sein.

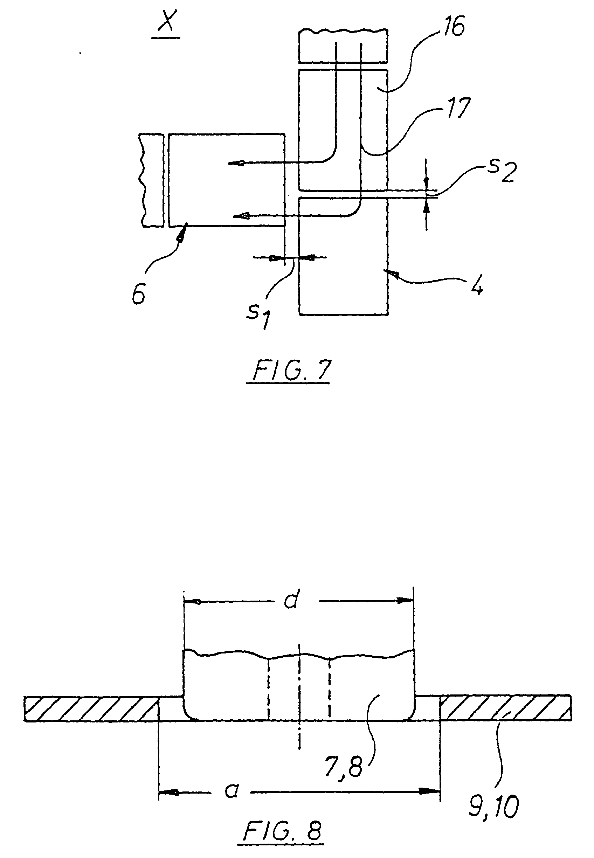

Wie dies insbesondere auch aus der Fig. 8 ersichtlich ist, sind die obere Magnetspule 9 und die untere Magnetspule 10 als Scheibenspulen ausgebildet mit einer oder mehreren Windungen, die schneckenförmig verlaufen und aus verseilten Kupfer-Litzendrähten hergestellt sind.

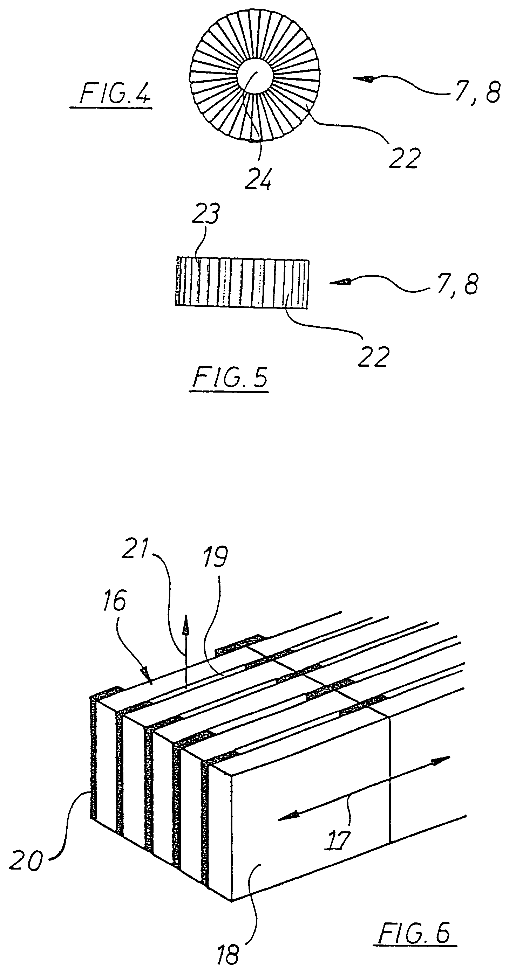

Der Fig. 8 ist ferner zu entnehmen, dass die Magnetspulen 9, 10 die Polschuhenden mit einem dazwischenliegenden und umlaufenden Magnetspule/Polschuh-Spalt (a) umfassen. Wie dies insbesondere aus der Fig. 4 ersichtlich ist, die eine Draufsicht auf einen der Polschuhe 7, 8 zeigt, sind die Polschuhe 7, 8 kreisrund ausgebildet. Der Magnetspule/Polschuh-Spalt (a) liegt in einem Größenbereich von 0,07 bis 0,1 mal dem Polschuhdurchmesser (d), wobei die Magnetspule in etwa flächenbündig mit der Polschuhendfläche angeordnet ist und die umlaufende Kante an der Polschuhendfläche abgerundet ist.

Des weiteren wird in Abhängigkeit vom Polschuhdurchmesser (d) auch die Größe des Befeldungsspaltes 12 ausgelegt, um die Streufelder zu reduzieren. So ist in einer bevorzugten Ausführungsform zur Vermeidung von Streufeldern der Polschuhdurchmesser (d) größer als der Befeldungsspalt 12.

Wie dies aus den Fig. 2 und 3 ersichtlich ist, die jeweils eine Seitenansicht bzw. eine Draufsicht auf das Magnetjoch 2 zeigen, ist das Magnetjoch 2 aus quaderförmigen Ferritbausteinen 16 zusammengesetzt, deren Oberflächen von Sinterschichten befreit und jeweils plan-parallel geschliffen sind. Diese quaderförmigen Ferritbausteine 16 sind wiederum, wie dies aus der Fig. 6 ersichtlich ist, aus aneinandergereihten, im Magnetjoch 2 längs der Magnetflussrichtung 17 ausgerichteten Ferritplatten 18 aufgebaut.

Diese Ferritplatten 18 sind quer zur Magnetflussrichtung 17 durch Isolations-/Kühlungs-Spalte 19 voneinander getrennt. In diese lsolations-/Kühlungs-Spalte 19 sind in Seitenbereichen Kunststoff-Separatoren 20 eingefügt, wobei die Ferritplatten 18 über diese Kunststoff-Separatoren 20 zu den quaderförmigen Ferritbausteinen 16 als Jochelemente verklebt sind. Durch die Isolations-/Kühlungs-Spalte 19 kann zur Kühlung des Magnetjochs 2 Kühlluft geleitet werden, wie dies in der Fig. 6 mit dem Pfeil 21 schematisch dargestellt ist.

Den Fig. 4 und 5 kann entnommen werden, dass die runden Polschuhe 7, 8 aus in der Draufsicht keilförmigen Ferritbausteinen 22 zusammengesetzt sind, deren Oberflächen ebenfalls von Sinterschichten befreit und plan-parallel geschliffen sind. Zwischen die keilförmigen Ferritbausteine 22 sind zur Bildung von lediglich schematisch dargestellten lsolations-/Kühlungs-Spalten 23 ebenfalls Separatoren eingefügt, über die benachbarte Ferritbausteine 22 miteinander verklebt sind. Die Separatoren sind in den schematischen Darstellungen der Fig. 4 und 5 nicht dargestellt.

Den Fig. 4 und 5 kann weiter entnommen werden, dass die Polschuhe 7, 8 eine axiale, rohrförmige Aussparung 24 aufweisen, durch die Kühlluft in den Magnetfeldapplikator 1 einleitbar ist, wie dies insbesondere auch aus der Fig. 1 ersichtlich ist.

In Fig. 7 ist dargestellt, dass die quaderförmigen Ferritbausteine 16 längs der Magnetflussrichtung 17 nur über einen sehr schmalen Anlagespalt (s₂) aneinanderliegen. Wie dies weiter aus der Fig. 7 ersichtlich ist, sind insbesondere an den Übergangsstellen zwischen dem relativ zum unteren Querjochteil 6 verstellbaren Vertikaljochteilen 3, 4 sowie an den Übergangsstellen zwischen den Querjochteilen 5, 6 und den Polschuhen 7, 8 zur vorteilhaften Steuerung des Magnetflusses Zwangsluftspalte (s₁) vorgesehen. Diese Zwangsluftspalte (s₁) weisen beispielsweise eine Spaltweite von 2 bis 3 mm auf und sind sehr groß im Vergleich zu den Änlagespalten (s₂).

Das Magnetfeld wird durch die Magnetspulen 9, 10 erzeugt, die mit einem Kondensator, der hier nicht dargestellt ist, zu einem Schwingkreis verbunden sind, in dem dann die Energie als Blindleistung mit der Resonanzfrequenz des Kreises pendelt. Die Magnetfeldstärken liegen vorzugsweise im Bereich von 1 bis 20 kA/m, während die Frequenzen im Bereich von vorzugsweise 20 bis 500 kHz liegen. Für eine Thermoseed-Anwendung des Magnetfeldapplikators riechen 1 bis 2 kA/m aus, während für Anwendungen mit magnetischen Flüssigkeiten höhere Feldstärken benötigt werden.

## Patentansprüche

1. Magnetfeldapplikator zur Aufheizung von magnetischen oder magnetisierbaren Substanzen oder Festkörpern in biologischem Gewebe,
mit einem Magnetjoch (2),
mit zwei gegenüberliegenden, durch einen Befeldungsspalt beabstandeten Polschuhen (7,8) am Magnetjoch, und
mit zwei jeweils einem Polschuh zugeordneten Magnetspulen (9,10) zur Erzeugung eines Magnetwechselfeldes,
**dadurch gekennzeichnet,**
**dass** das Magnetjoch (2) und die Polschuhe (7, 8) aus zusammenmontierten Ferritbausteinen (16, 22) bestehen, und
**dass** das Magnetjoch (2) in einer M-Form als Drei-Schenkel-Anordnung ausgebildet ist mit zwei beabstandeten, parallelen Vertikaljochteilen (3, 4) vorzugsweise gleicher Geometrie und mit zwei dazwischen angeschlossenen Querjochteilen (5, 6) mit jeweils in deren Mitte angeordneten und aufeinander zugerichteten Polschuhen (7, 8), wovon wenigstens ein Querjochteil (6) mit angeschlossenem Polschuh (8) und zugeordneter Magnetspule (10) als Baueinheit relativ zum anderen Querjochteil (5) zur Einstellung der Befeldungsspaltweite verstellbar ist.

2. Magnetfeldapplikator nach Anspruch 1, **dadurch gekennzeichnet,**
**dass** eine Baueinheit aus einem unteren Querjochteil (6) und dem zugeordneten Polschuh (8) mit Magnetspule (10) ortsfest angebracht ist, und
**dass** demgegenüber ein Portal aus den beiden Vertikaljochteilen (3, 4) und aus dem angeschlossenen oberen Querjochteil (5) und dem zugeordneten Polschuh (7) mit Magnetspule (9) mittels einer Vertikalverstelleinrichtung (11) zur Einstellung der Befeldungsspaltweite verstellbar ist.

3. Magnetfeldapplikator nach Anspruch 2, **dadurch gekennzeichnet, dass** die Vertikalverstelleinrichtung aus wenigstens einem motorisch steuerbaren, von unten her an den Vertikaljochteilen (3, 4) angreifenden Linearantrieb, insbesondere aus einem selbsthemmenden Spindelantrieb (11), besteht.

4. Magnetfeldapplikator nach einem der Ansprüche 1 bis 3, **dadurch gekennzeichnet, dass** das Magnetjoch (2) in einer Tragkonstruktion gehalten ist, in der zudem Kühlluft zuführbar und leitbar ist.

5. Magnetfeldapplikator nach einem der Ansprüche 1 bis 4, **dadurch gekennzeichnet, dass** der Befeldungsspalt (12) jeweils seitlich zu den Vertikaljochteilen (3, 4) hin durch Fefdbegrenzugsspulen und/oder durch einen Einschubraum umgrenzende, gegebenenfalls gegeneinander vertikal verstellbare Schottwände (14, 15) begrenzt ist.

6. Magnetfeldapplikator nach einem der Ansprüche 1 bis 5, **dadurch gekennzeichnet, dass** das zu befeldende Gewebe ein Tumorbereich eines Patienten ist, in das eine magnetische Flüssigkeit mit vorzugsweise magnetischen und/oder magnetisierbaren Nanoteilchen einbringbar ist, wobei der Tumorbereich auf Temperaturwerte vorzugsweise über ca. 41°C lokal erhitzbar ist.

7. Magnetfeldapplikator nach Anspruch 6, **dadurch gekennzeichnet, dass** Magnetwechselfelder mit Magnetfeldstärken von vorzugsweise 10 bis 15 kA/m und Frequenzen von vorzugsweise 50 bis 100 kHz verwendet werden.

8. Magnetfeldapplikator nach einem der Ansprüche 1 bis 7, **dadurch gekennzeichnet, dass** die Magnetspulen (9, 10) Scheibenspulen mit wenigstens einer schneckenförmig verlaufenden Windung sind, die jeweils einem Polschuh (7, 8) zugeordnet sind und das jeweilige Polschuhende mit einem dazwischenliegenden umlaufenden Magnetspule/Polschuh-Spalt (a) umfassen.

9. Magnetfeldapplikator nach Anspruch 8, **dadurch gekennzeichnet, dass** die Magnetspulen (9, 10) eine oder mehrere Windungen aufweisen, die schneckenförmig verlaufen und die aus verseilten Kupfer-Litzendrähten hergestellt sind.

10. Magnetfeldapplikator nach Anspruch 8 oder Anspruch 9, **dadurch gekennzeichnet,**
**dass** die Polschuhe (7, 8) in einer Draufsicht kreisrund ausgebildet sind und mit gegeneinander weisenden, parallel ausgerichteten Polschuhkreisflächen im Abstand des Befeldungsspalts (12) gegenüberliegen, und
**dass** die Magnetspulen (9, 10) entsprechend kreisringförmig ausgebildet sind.

11. Magnetfeldapplikator nach einem der Ansprüche 8 bis 10, **dadurch gekennzeichnet,**
**dass** der Magnetspule/Polschuh-Spalt (a) in einem Größenbereich von 0,07 bis 0,1 mal dem Polschuhdurchmesser (d) liegt, und
**dass** die Magnetspule (9, 10) etwa flächenbündig bezüglich der Polschuhendfläche angeordnet ist, wobei die umlaufende Kante an der Polschuhendfläche abgerundet ist.

12. Magnetfeldapplikator nach einem der Ansprüche 1 bis 11, **dadurch gekennzeichnet, dass** der Polschuhdurchmesser (d) größer als der Befeldungsspalt (12) ist.

13. Magnetfeldapplikator nach einem der Ansprüche 1 bis 12, **dadurch gekennzeichnet,**
**dass** das Magnetjoch (2) aus quaderförmigen Ferritbausteinen (16) zusammengesetzt ist, deren Flächen gegebenenfalls von Sinterschichten befreit und plan-parallel geschliffen sind, und
**dass** runde Polschuhe (7, 8) aus entsprechend bearbeiteten, in der Draufsicht keilförmigen Ferritbausteinen (22) zusammengesetzt sind.

14. Magnetfeldapplikator nach einem der Ansprüche 1 bis 13, **dadurch gekennzeichnet, dass** quaderförmige Ferritbausteine (16) aus aneinandergereihten, im Magnetjoch (2) längs des Magnetflusses ausgerichteten Ferritplatten (18) bestehen, die quer zum Magnetfluss durch Isolations-/Kühlungs-Spalte (19) voneinander getrennt sind, durch die Kühlluft leitbar ist und die längs des Magnetflusses über einen nur sehr schmalen Anlagespalt (s₂) aneinanderliegen.

15. Magnetfeldapplikator nach Anspruch 14, **dadurch gekennzeichnet, dass** in die Isolations-/Kühlungs-Spalte (19) vorzugsweise in Seitenbereichen Separatoren (20) aus Kunststoff eingefügt sind und die Ferritplatten (18) über die Separatoren (20) zu Ferritbausteinen als Jochelemente verklebt sind.

16. Magnetfeldapplikator nach Anspruch 13, **dadurch gekennzeichnet,**
**dass** zwischen keilförmigen Ferritbausteinen (22) zur Bildung von Isolations-/Kühlungs-Spalten (23) Separatoren eingefügt sind, über die benachbarte Ferritbausteine gegebenenfalls miteinander verklebt sind, und
**dass** eine axiale, rohrförmige Aussparung (24) zur Bildung jeweils eines rohrförmigen Polschuhs (7, 8) vorgesehen ist, durch die Kühlluft einleitbar ist.

17. Magnetfeldapplikator nach einem der Ansprüche 1 bis 16, **dadurch gekennzeichnet, dass** an toleranzbehafteten Übergangsstellen zwischen Magnetjochbausteinen, insbesondere an Übergangsstellen zwischen relativ zueinander verstellbaren Querjochteilen (6) und Vertikaljochteilen (3, 4) und/oder an Übergangsstellen zwischen Querjochteilen (5, 6) und den Polschuhen (7, 8) zur Steuerung des Magnetflusses Zwangsluftspalte (s₁) vorzugsweise mit einer Spaltweite von 2 bis 3 mm vorgesehen sind, wobei die Spaltweiten dieser Zwangsluftspalte (s₁) sehr groß im Vergleich zu den Anlagespalten (s₂) insbesondere im Querjochverstellbereich sind.

## Claims

1. Magnetic field applicator for heating magnetic or magnetizable substances or solids in biological tissue,
comprising a magnetic yoke (2),
with two pole shoes (7, 8) facing each other and separated by a prescribed distance defining the magnetic field exposure volume, and
comprising two magnetic coils (9, 10) respectively assigned to a pole shoe for the production of an alternating magnetic field,
**characterized in that**
the magnetic yoke (2) and the pole shoes (7, 8) are composed of ferrite segments (16, 22) assembled together, and
**in that** the magnetic yoke (2) is formed in an M shape as a three-leg arrangement, with two spaced-apart, parallel vertical yoke parts (3, 4), preferably of the same geometry and with two transverse yoke parts (5, 6) connected in between, with pole shoes (7, 8) arranged in each case in the centre thereof and directed at one another, of which at least one transverse yoke part (6) with connected pole shoe (8) and assigned magnetic coil (10) is adjustable as a structural unit in relation to the other transverse yoke part (5) for setting the gap defining the magnetic field exposure volume.

2. Magnetic field applicator according to Claim 1, **characterized**
**in that** a structural unit comprising a lower transverse yoke part (6) and the assigned pole shoe (8) with magnetic coil (10) is fixed in place, and
**in that** a portal comprising the two vertical yoke parts (3, 4) and comprising the connected upper transverse yoke part (5) and the assigned pole shoe (7) with magnetic coil (9) is adjustable with respect to the said structural unit by means of a vertical adjusting device (11) for setting the gap defining the magnetic field exposure volume.

3. Magnetic field applicator according to Claim 2, **characterized in that** the vertical adjusting device is composed of at least one motor-controlled linear drive acting from below on the vertical yoke parts (3, 4), in particular of a self-locking spindle drive (11).

4. Magnetic field applicator according to one of Claims 1 to 3, **characterized in that** the magnetic yoke (2) is held in a support assembly, in which moreover cooling air can be supplied and passed.

5. Magnetic field applicator according to one of Claims 1 to 4, **characterized in that** the gap defining the magnetic field exposure volume (12) is laterally limited in each case with respect to the vertical yoke parts (3, 4) by field limitation coils and/or by bulkheads (14, 15) which bound a pushing-in space and may be vertically adjustable with respect to one another.

6. Magnetic field applicator according to one of Claims 1 to 5, **characterized in that** the tissue exposed to the field is a tumour zone of a patient into which a magnetic fluid with preferably magnetic and/or magnetizable nano-particles has been introduced, the tumour zone being locally heatable to temperature values of preferably over about 41°C.

7. Magnetic field applicator according to Claim 6, **characterized in that** alternating magnetic fields with magnetic field strengths of preferably 10 to 15 kA/m and frequencies of preferably 50 to 100 kHz are used.

8. Magnetic field applicator according to one of Claims 1 to 7, **characterized in that** the magnetic coils (9, 10) are disc-shaped coils with at least one helicoidally extending winding, which are respectively assigned to a pole shoe (7, 8) and enclose the respective pole shoe end with a peripheral magnetic-coil/pole-shoe gap (a) lying in between.

9. Magnetic field applicator according to Claim 8, **characterized in that** the magnetic coils (9, 10) have one or more windings which extend helicoidally and are produced from stranded copper wires.

10. Magnetic field applicator according to Claim 8 or 9, **characterized**
**in that** the pole shoes (7, 8) are of a circular form in plan view and lie with mutually facing, parallel aligned circular pole shoe surfaces spaced apart by the distance defining the magnetic field exposure volume (12), and
**in that** the magnetic coils (9, 10) are of a correspondingly circular ring-shaped form.

11. Magnetic field applicator according to one of Claims 8 to 10, **characterized**
**in that** the magnetic-coil/pole-shoe gap (a) lies in a size range of 0.07 to 0.1 times the pole shoe diameter (d), and
**in that** the magnetic coil (9, 10) is arranged approximately flush with the pole shoe end face, the peripheral edge being rounded off on the pole shoe end face.

12. Magnetic field applicator according to one of Claims 1 to 11, **characterized in that** the pole shoe diameter (d) is greater than the distance defining the magnetic field exposure volume (12).

13. Magnetic field applicator according to one of Claims 1 to 12, **characterized**
**in that** the magnetic yoke (2) is composed of cuboidal ferrite segments (16), the surfaces of which are ground free of any sintering layers and such that they are plane-parallel, and
**in that** round pole shoes (7, 8) are composed of correspondingly machined ferrite segments (22) which are wedge-shaped in plan view.

14. Magnetic field applicator according to one of Claims 1 to 13, **characterized in that** cuboidal ferrite segments (16) are composed of ferrite plates (18) aligned in rows along the magnetic flow in the magnetic yoke (2) and are separated from one another transversely to the magnetic flow by isolating/cooling gaps (19), through which cooling air can be passed and which lie adjacent to one another along the magnetic flow with only a narrow abutment gap (s₂).

15. Magnetic field applicator according to Claim 14, **characterized in that** separators (20) of plastic are inserted into the isolating/cooling gaps (19), preferably in side regions, and the ferrite plates (18) are adhesively bonded via the separators (20) to form ferrite segments as yoke elements.

16. Magnetic field applicator according to Claim 13, **characterized**
**in that**, to form isolating/cooling gaps (23), separators are inserted between wedge-shaped ferrite segments (22) and may be used if appropriate for the adhesive bonding of adjacent ferrite segments to one another, and
**in that** an axial, tubular opening (24) is provided for respectively forming a tubular pole shoe (7, 8) and through which cooling air can be passed.

17. Magnetic field applicator according to one of Claims 1 to 16, **characterized in that** forced-air gaps (s₁), preferably with a gap width of 2 to 3 mm, are provided at tolerance-affected transition points between magnetic yoke segments, in particular at transition points between transverse yoke parts (6) and vertical yoke parts (3, 4) which are adjustable in relation to one another and/or at transition points between transverse yoke parts (5, 6) and the pole shoes (7, 8) to control the magnetic flow, the gap widths of these forced-air gaps (s₁) being very large in comparison with the abutment gaps (s₂), in particular in the transverse yoke adjusting region.

## Revendications

1. Applicateur de champ magnétique pour le chauffage de substances ou de corps solides magnétiques ou magnétisables dans des tissus biologiques,
présentant une carcasse d'aimant (2)
avec deux épanouissements polaires (7, 8) situés sur la carcasse d'aimant qui sont espacés par un entrefer et en vis-à-vis, et
ayant deux bobines d'excitation (9, 10) qui sont affectées chacune à un épanouissement polaire et destinées à la génération d'un champ magnétique alternant,
**caractérisé**
**en ce que** la carcasse d'aimant (2) et les épanouissements polaires (7, 8) sont constitués d'éléments de ferrite (16, 22) assemblés, et
**en ce que** la carcasse d'aimant (2) présente une forme en M sous forme d'une disposition à trois branches comprenant deux parties verticales de carcasse (3, 4) parallèles, écartées et présentant les mêmes propriétés géométriques, et deux parties transversales de carcasse (5, 6) connectées entre les premières et présentant, dans leur centre, des épanouissements polaires (7, 8) dirigés l'un vers l'autre, au moins une partie transversale de carcasse (6) et son épanouissement polaire (8) connecté ainsi qu'une bobine d'excitation (10) correspondante étant, en tant que composant, ajustable par rapport à l'autre partie transversale de carcasse (5), afin de régler l'écartement de l'entrefer.

2. Applicateur de champ magnétique selon la revendication 1, **caractérisé**
**en ce que** l'on monte de manière fixe un composant constitué d'une partie transversale inférieure de carcasse (6) et l'épanouissement polaire (8) correspondant avec sa bobine d'excitation (10), et
**en ce que** l'on peut ajuster, par rapport à ce composant, un portique constitué des deux parties verticales de carcasse (3, 4) et de la partie transversale de carcasse supérieure connectée (5) ainsi que l'épanouissement polaire (7) correspondant avec sa bobine d'excitation (9), au moyen d'un dispositif d'ajustement vertical (11) destiné au réglage de l'écartement de l'entrefer.

3. Applicateur de champ magnétique selon la revendication 2, **caractérisé en ce que** le dispositif d'ajustage vertical est constitué d'au moins un mécanisme d'entraînement linéaire entraînable par moteur, qui saisit les parties verticales de carcasse (3, 4) par le bas, en particulier d'un entraînement à broche autobloquant (11).

4. Applicateur de champ magnétique selon l'une quelconque des revendications 1 à 3, **caractérisé en ce que** la carcasse d'aimant (2) est logée dans un bâti qui permet, de plus, l'introduction et la circulation de l'air de refroidissement.

5. Applicateur de champ magnétique selon l'une quelconque des revendications 1 à 4, **caractérisé en ce que** l'entrefer (12) est limité par des couronnes limitatives de champ, de part et d'autre des parties verticales de carcasse (3, 4), et/ou par des cloisons étanches (14, 15) qui délimitent un espace d'insertion et qui sont éventuellement déplaçables, l'une par rapport à l'autre, dans le sens vertical.

6. Applicateur de champ magnétique selon l'une quelconque des revendications 1 à 5, **caractérisé en ce que** le tissu à exposer au champ est une zone de tumeur d'un patient dans laquelle on peut introduire un liquide magnétique présentant de préférence des nanoparticules magnétiques et/ou magnétisables, la zone de tumeur pouvant être chauffée localement à une température de préférence supérieure à 41°C environ.

7. Applicateur de champ magnétique selon la revendication 6, **caractérisé en ce que** l'on applique un champ magnétique alternatif présentant une intensité du champ magnétique comprise de préférence entre 10 kA/m et 15 kA/m et une fréquence comprise de préférence entre 50 kHz et 100 kHz.

8. Applicateur de champ magnétique selon l'une quelconque des revendications 1 à 7, **caractérisé en ce que** les bobines d'excitation (9, 10) sont sous forme de galettes présentant au moins une spire de forme hélicoïdale, qui sont assignées chacune à un épanouissement polaire (7, 8) et qui entourent l'extrémité de l'épanouissement polaire correspondant avec une lumière périphérique entre la bobine d'excitation et l'épanouissement polaire (a).

9. Applicateur de champ magnétique selon la revendication 8, **caractérisé en ce que** les bobines d'excitation (9, 10) présentent une ou plusieurs spires de forme hélicoïdale préparées à partir de fil de cuivre en toron torsadé.

10. Applicateur de champ magnétique selon la revendication 8 ou 9, **caractérisé**
**en ce que** les épanouissements polaires (7, 8) sont façonnées en forme circulaire, en vue de dessus, et sont en vis-à-vis, de sorte que les surfaces circulaires d'épanouissement polaire sont disposées parallèlement et en vis-à-vis l'une par rapport l'autre, séparées par l'entrefer (12), et
**en ce que** les bobines d'excitation (9, 10) sont façonnées en forme circulaire, en correspondance.

11. Applicateur de champ magnétique selon l'une quelconque des revendications 8 à 10, **caractérisé**
**en ce que** la lumière entre la bobine d'excitation et l'épanouissement polaire (a) présente une taille comprise entre 0,07 fois et 0,1 fois du diamètre (d) de l'épanouissement polaire, et
**en ce que** la bobine d'excitation (9, 10) est disposée approximativement en affleurement par rapport à la surface de l'extrémité de l'épanouissement polaire, l'arête périphérique de la surface de l'extrémité de l'épanouissement polaire étant arrondie.

12. Applicateur de champ magnétique selon l'une quelconque des revendications 1 à 11, **caractérisé en ce que** le diamètre (d) de l'épanouissement polaire est plus grand que l'entrefer (12).

13. Applicateur de champ magnétique selon l'une quelconque des revendications 1 à 12, **caractérisé**
**en ce que** la carcasse d'aimant (2) est composée d'éléments de ferrite (16) parallélépipédiques dont les surfaces sont éventuellement libérées de couches frittées et qu'elles sont rectifiées pour obtenir des faces planes et parallèles, et
**en ce que** les épanouissements polaires (7, 8) ronds sont composés d'éléments de ferrite (22) qui présentent, vus de dessus, une forme cunéiforme, et que l'on a traité de manière similaire.

14. Applicateur de champ magnétique selon l'une quelconque des revendications 1 à 13, **caractérisé en ce que** les éléments de ferrite (16) parallélépipédiques sont constitués de plaques de ferrite (18) alignées l'une derrière l'autre, disposées dans la carcasse d'aimant (2) et dirigées le long du flux magnétique, qui sont séparées l'une de l'autre par une fente d'isolation/de refroidissement (19) située de manière transversale par rapport au flux magnétique et permettant une circulation de l'air de refroidissement, et qui sont disposées l'une contre l'autre, dans le sens longitudinal du flux magnétique, en présentant seulement une discontinuité de contact (s₂) étroite.

15. Applicateur de champ magnétique selon la revendication 14, **caractérisé en ce que** l'on introduit dans la fente d'isolation/de refroidissement (19), de préférence dans ses zones latérales, des séparateurs (20) constitués de matière synthétique, et **en ce que** l'on colle les plaques de ferrite (18) par l'intermédiaire des séparateurs (20) afin d'obtenir les éléments de ferrite en tant qu'éléments de carcasse.

16. Applicateur de champ magnétique selon la revendication 13, **caractérisé**
**en ce que** l'on insère des séparateurs entre les éléments de ferrite (22) cunéiformes afin de former des fentes d'isolation/de refroidissement (23), séparateurs qui servent éventuellement à coller des éléments de ferrite voisins afin d'obtenir un épanouissement polaire, et
**en ce que** l'on prévoit une ouverture (24) axiale tubulaire afin de former à chaque fois un épanouissement polaire (7, 8) tubulaire, ouverture (24) qui permet l'introduction de l'air de refroidissement.

17. Applicateur de champ magnétique selon l'une quelconque des revendications 1 à 16, **caractérisé en ce que** des discontinuités magnétiques obligatoires (s₁) sont prévues, qui présentent de préférence un interstice allant de 2 mm à 3 mm, et qui sont situées au niveau des zones de transition à tolérance entre les éléments de carcasse d'aimant, en particulier au niveau des zones de transition entre les parties transversales de carcasse (6) que l'on peut ajuster l'une par rapport à l'autre et les parties verticales de carcasse (3, 4) et/ou au niveau des zones de transition entre les parties transversales de carcasse (5, 6) et les épanouissements polaires (7, 8), afin de réguler le flux magnétique, les interstices de ces discontinuités magnétiques obligatoires (s₁) étant très grands par rapport aux discontinuités de contact (s₂), en particulier dans la zone d'ajustement des parties transversales de la carcasse.
